# EUROPEAN PATENT APPLICATION

(11) **EP 2 213 261 A1**
(43) Date of publication of application: **04.08.2010**
(21) Application number: 09014992.3
(22) Date of filing: 03.12.2009
(51) Int. Cl.: A61F 2/34, A61F 2/46

(54) **Tool for implanting acetabular cup with external screw retention features**

(30) Priority: 29.01.2009 US 361675
(71) Applicant: Zimmer GmbH, 8404 Winterthur (CH)
(72) Inventor: Howald, Ralph, 9230 Flawil (CH); Breimesser, Hermann, 8353 Elgg (CH)
(74) Representative: Manitz, Finsterwald & Partner GbR

(57) **Abstract**

A tool (40) is provided for stabilizing acetabular cup (10) in pelvis P during securement. Tool (40) includes shaft (42), handle (44) coupled to an end of shaft (42), and impactor or projection (45) coupled to shaft (42) at an end opposite of handle (44).
Tool (40) further includes at least one pin (46) coupled to shaft (42). In the illustrated embodiment, tool (40) includes two pins (46) extending in parallel. As pins (46) are moved into a locking position, pins (46) extend substantially transversely, or non- parallel, to shaft (42). Each pin (46) may include a sharp, pointed end (47), that is configured to be driven into bone. For example, end (47) of each pin (46) may include a pointed Kirschner wire or another suitable device that is configured to be driven into bone.

## Description

### BACKGROUND

### 1. Field of the Invention.

The present invention relates to orthopaedic implants. More particularly, the present invention relates to a tool and method for implanting an acetabular cup of the type used in a hip arthroplasty procedure, for example.

### 2. Description of the Related Art.

Orthopaedic implants are commonly used to replace some or all of a patient's hip joint to restore or increase use of the hip joint following a traumatic injury or deterioration due to aging or illness, for example. During a hip replacement procedure, a prosthetic femoral component may be used to replace a portion of the patient's femur and a prosthetic acetabular component may be used to replace a portion of the patient's pelvis. The femoral component may include a stem portion, a neck portion, and a head portion. The stem portion may be positioned within the prepared femoral canal of the patient's femur and secured via bone cement or by a press-fit. The neck portion extends between the stem portion and the head portion. The head portion articulates within the prosthetic acetabular component, and may be constructed of metal or ceramic, for example.

Known prosthetic acetabular components generally include a cup portion and a liner portion, each having a substantially hemispherical shape. The cup portion may be constructed of metal and may be implanted by inserting one or more screws through holes in the hemispherical inner surface of the cup and into the surrounding bone of the patient's pelvis. After the cup portion is implanted, the liner portion, which may be constructed of a polymer, is fitted in place within the metal cup portion by suitable mechanical fasteners or by a snap-fit engagement, for example. The polymer liner portion then receives the metal head portion of the prosthetic femoral component, to provide "metal-on-poly" articulation.

Other known prosthetic acetabular components generally include a "monoblock" cup, which is constructed substantially entirely of metal. Such cups include metallic articulating surfaces for receiving the metal or ceramic head portion of the prosthetic femoral component, to provide "metal-on-metal" or "ceramic-on-metal" articulation.

### SUMMARY

The present invention provides a tool and method for implanting an acetabular cup having external screw retention features.

According to an embodiment of the present invention, an orthopaedic tool is provided for implanting a prosthetic socket. The prosthetic socket includes a substantially hemispherical body having a convex exterior surface defining an outer periphery of the prosthetic socket, a concave interior articulating surface, and a rim extending between the convex exterior surface and the concave interior articulating surface near a top portion of the prosthetic socket. The orthopaedic tool includes a shaft configured to couple to the prosthetic socket and a first pin having an end. The first pin is coupled to the shaft for movement relative to the shaft above the rim of the prosthetic socket from a first position in which the end of the first pin is located within the outer periphery of the prosthetic socket to a second position in which the end of the first pin is located beyond the outer periphery of the prosthetic socket.

According to another embodiment of the present invention, an orthopaedic system is provided including a prosthetic socket and a tool. The prosthetic socket includes a substantially hemispherical body that defines a polar region and an equatorial region located above the polar region. The body of the prosthetic socket includes a convex exterior surface that defines an outer periphery of the prosthetic socket, a concave interior articulating surface, and a longitudinal axis. The tool includes a shaft configured to couple to the prosthetic socket and a first pin having an end. The first pin is coupled to the shaft for movement relative to the shaft above the equatorial region of the prosthetic socket from a first position in which the end of the first pin is located within the outer periphery of the prosthetic socket to a second position in which the end of the first pin is located beyond the outer periphery of the prosthetic socket.

According to yet another embodiment of the present invention, a method is provided for implanting a prosthetic socket. The prosthetic socket includes a substantially hemispherical body that defines a polar region and an equatorial region located above the polar region, and the body includes a convex exterior surface, a concave interior articulating surface, and at least one screw retention element. The method includes the steps of positioning the convex exterior surface of the prosthetic socket against a bone, and inserting a first pin of a tool into a first location of the bone above the equatorial region of the prosthetic socket and adjacent to the prosthetic socket.

The tool according to the invention can be used in an method of implanting a prosthetic socket, the prosthetic socket including a substantially hemispherical body that defines a polar region and an equatorial region located above the polar region, the body including a convex exterior surface, a concave interior articulating surface, and at least one screw retention element, the method comprising the steps of:
positioning the convex exterior surface of the prosthetic socket against a bone; and
inserting a first pin of the tool into a first location of the bone above the equatorial region of the prosthetic socket and adjacent to the prosthetic socket.
The first location of the bone can be located radially opposite from the at least one screw retention element.
The tool can further include a shaft coupled to the first pin, and the method can further comprise the step of forcing the shaft of the tool against the prosthetic socket during the inserting step.
The tool can further include a shaft coupled to the first pin, and the inserting step can further comprise at least one of rotating and translating the first pin relative to the shaft.
The positioning step can comprise positioning the prosthetic socket against one of a pelvis and a scapula.
The method can comprise the step of screwing a first screw through the at least one screw retention element and into the bone.
The method can comprise the steps of: removing the first pin from the bone; inserting the first pin into a second location of the bone; and screwing a second screw into the bone.

### BRIEF DESCRIPTION OF THE DRAWINGS

The above-mentioned and other features and advantages of this invention, and the manner of attaining them, will become more apparent and the invention itself will be better understood by reference to the following description of embodiments of the invention taken in conjunction with the accompanying drawings, wherein:

Figure 1 is a top plan view of an embodiment of an acetabular cup implanted within the acetabulum of a patient's pelvis;

Figure 2 is a cross-sectional elevational view of the acetabular cup of Figure 1 implanted within the acetabulum of a patient's pelvis;

Figure 3 is a view similar to Figure 2, also showing an embodiment of a tool of the present invention;

Figure 3A is a cross-sectional view of the tool of Figure 3, taken along line 3A-3A of Figure 3;

Figure 4 is a view similar to Figure 3 showing another embodiment of a tool of the present invention; and

Figure 4A is a cross-sectional view of the tool of Figure 4, taken along line 4A-4A of Figure 4.

Corresponding reference characters indicate corresponding parts throughout the several views. The exemplifications set out herein illustrate exemplary embodiments of the invention and such exemplifications are not to be construed as limiting the scope of the invention in any manner.

### DETAILED DESCRIPTION

Orthopaedic implants of the type used in hip arthroplasty procedures, such as prosthetic femoral hip stems and acetabular cups, may be implanted according to surgical techniques described in U.S. Patent No. 6,676,706, issued January 13, 2004; U.S. Patent No. 6,860,903, issued March 1, 2005; U.S. Patent No. 6,953,480, issued October 11, 2005; U.S. Patent No. 6,991,656, issued January 31, 2006; abandoned U.S. Patent Application Serial No. 10/929,736, filed August 30, 2004; abandoned U.S. Patent Application Serial No. 10/952,301, filed September 28, 2004; currently pending U.S. Patent Application Serial No. 11/235,286, filed September 26, 2005; and currently pending U.S. Patent Application Serial No. 11/105,080, filed April 13, 2005, all titled METHOD AND APPARATUS FOR PERFORMING A MINIMALLY INVASIVE TOTAL HIP ARTHROPLASTY and all assigned to the assignee of the present application, the disclosures of which are hereby expressly incorporated herein by reference.

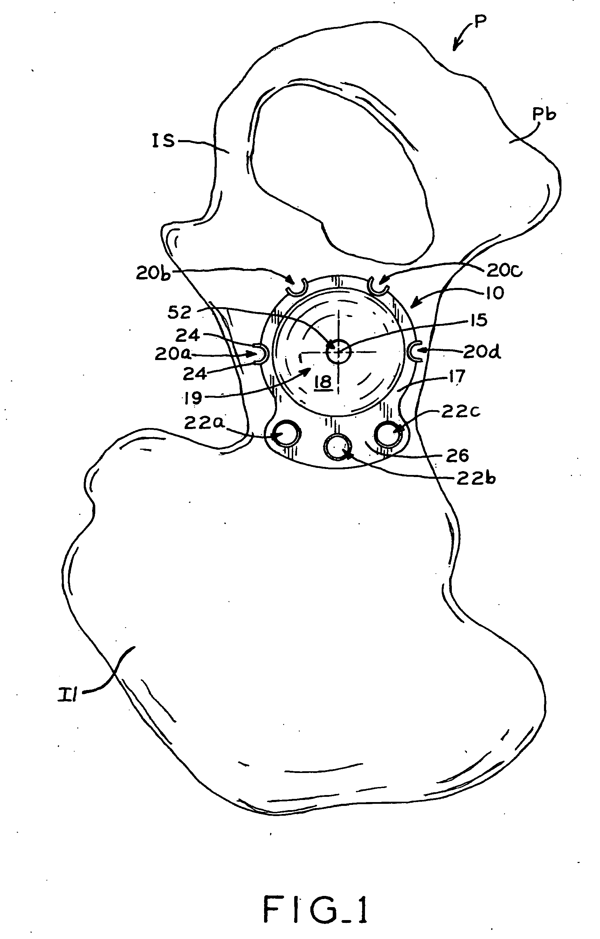

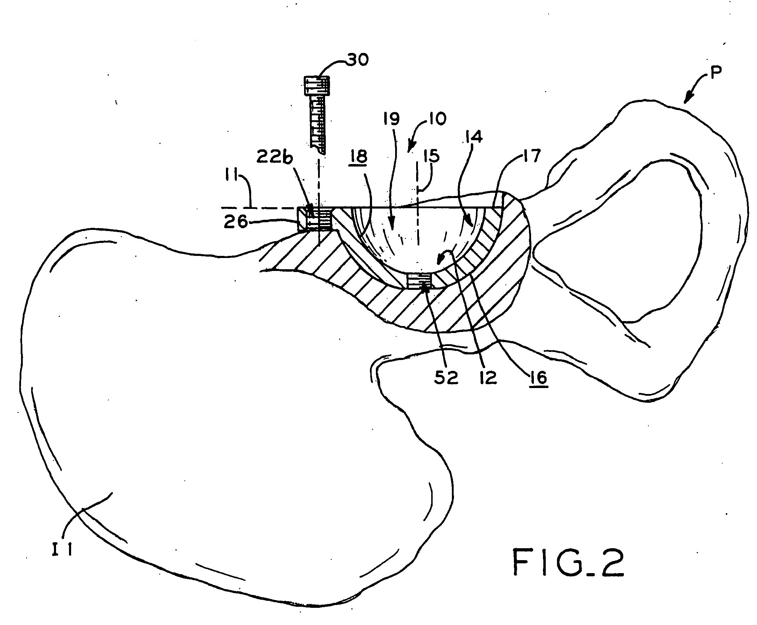

Referring to Figures 1 and 2, a portion of pelvis P of a patient is shown, including the ilium Il, ischium Is, and pubis Pb, of pelvis P. A prosthetic component, illustrated as acetabular cup 10, is implanted to replace a portion of the patient's pelvis P. Exemplary acetabular cups are described in U.S. Provisional Patent Application Serial No. 61/048,696, filed April 29, 2008, titled METAL ACETABULAR CUP WITH EXTERNAL SCREW RETENTION FEATURES, and assigned to the assignee of the present application, the disclosure of which is hereby expressly incorporated herein by reference. While the prosthetic component is described and depicted herein as being an acetabular cup of the type used in a hip arthroplasty procedure, the prosthetic component may be a glenoid component of the type used in a shoulder arthroplasty procedure or another prosthetic component, for example. Exemplary glenoid components are described in U.S. Patent Application Publication No. 2005/0261775, filed May 3, 2005, titled GLENOID ANCHOR, and U.S. Patent Application Publication No. 2008/0294268, filed January 17, 2006, titled BASE PLATFORM FOR AN ARTIFICIAL JOINT, both assigned to the assignee of the present application, the disclosures of which are hereby expressly incorporated herein by reference.

Acetabular cup 10 may be constructed entirely or substantially entirely of a suitable metal, such as titanium, a titanium alloy, or a cobalt-chromium-molybdenum alloy, for example. As shown in Figure 2, acetabular cup 10 is generally hemispherical in shape, having a body that is located substantially beneath equatorial plane 11, which may also be referred to as an entry plane. Acetabular cup 10 includes polar region 12 and equatorial region 14 located near equatorial plane 11. Acetabular cup 10 also includes longitudinal axis 15 that extends through the center of acetabular cup 10, from polar region 12 to equatorial region 14 of acetabular cup 10, in a direction essentially perpendicular to equatorial plane 11. For purposes of discussion, longitudinal axis 15 is assumed to generally coincide with the center of gravity of acetabular cup 10, although flange 26 of acetabular cup 10 may skew the center of gravity away from longitudinal axis 15.

Acetabular cup 10 also includes a convex, dome-shaped exterior bone-contacting surface 16, and a concave, hemispherical interior articulating surface 18, and rim 17 that extends between bone-contacting surface 16 and articulating surface 18. Articulating surface 18 may be constructed entirely or substantially entirely of a suitable metal. The concave articulating surface 18 defines socket 19 that may be configured to receive the head portion of a prosthetic femoral component (not shown) or a polymeric bearing liner (not shown), for example. As shown in Figure 2, the thickness of acetabular cup 10, or the distance between bone-contacting surface 16 and articulating surface 18 of acetabular cup 10, may be approximately 2 millimeters (mm), 3 mm, 4 mm, 5 mm, or more, for example.

Acetabular cup 10 further includes a plurality of external screw retention elements 20, 22, disposed about the periphery of acetabular cup 10 and radially offset from longitudinal axis 15. As used herein, "screw retention elements" are elements against which a bone fixation screw, such as screw 30, can act to secure acetabular cup 10 to pelvis P. The bone fixation screw may be a self-tapping screw, or alternatively, a non-self tapping screw inserted through pilot holes formed by a suitable tap. Also, as used herein, "external" refers to a location outside the concave, interior articulating surface 18 of acetabular cup 10, against which a bearing liner or femoral head may be positioned. Thus, as shown in Figure 1, external screw retention elements 20, 22, are located in equatorial region 14 of acetabular cup 10, outside of articulating surface 18. The location of screw retention elements 20, 22, outside of articulating surface 18 of acetabular cup 10 obviates disruptions in the smooth, articulating surface 18 while providing secure anchoring of acetabular cup 10 to pelvis P, allowing acetabular cup 10 to be constructed entirely or substantially entirely of metal.

Screw retention elements 20, 22, may be configured to receive various types of bone fixation screws 30. For example, the shaft of bone fixation screw 30 may extend between fins 24 of screw retention elements 20a, 20b, 20c, 20d, with the head of screw 30 resting against fins 24. Screw retention elements 20a, 20b, 20c, 20d, may be used to orient bone fixation screw 30 at a desired angular orientation with respect to acetabular cup 10. As another example, screw retention elements 22a, 22b, 22c, may extend through flange 26 that projects from equatorial region 14 of acetabular cup 10. Screw retention elements 22a, 22b, 22c, may be threaded to engage a threaded head of bone fixation screw 30, for example. Screw retention elements 22a, 22b, 22c, may be used to locate bone fixation screw 30 in a fixed angular orientation with respect to acetabular cup 10.

The location of the screw retention elements 20, 22, may be selected to correspond with desired areas of pelvis P. According to an exemplary embodiment of the present invention, bone fixation screws 30 may be inserted through screw retention elements 20, 22, and screwed into desired areas of pelvis P that have adequate bone stock for anchoring acetabular cup 10 to pelvis P. For example, in the illustrated embodiment of Figures 1 and 2, screw retention element 20a is positioned to receive screw 30 between ilium Il and ischium Is of pelvis P; screw retention element 20b is positioned to receive screw 30 in ischium Is of pelvis P; screw retention element 20c is positioned to receive screw 30 in pubis Pb of pelvis P; and screw retention element 20d is positioned to receive screw 30 between pubis Pb and ilium Il of pelvis P. Also, in the illustrated embodiment of Figures 1 and 2, flange 26 and screw retention elements 22a, 22b, 22c, are positioned to receive multiple screws 30 in ilium Il of pelvis P.

In use, the acetabulum of pelvis P is prepared, such as by reaming. Then, acetabular cup 10 is placed within the prepared acetabulum to align screw retention elements 20, 22, with the desired anatomical landmarks of pelvis P, as described above. For example, acetabular cup 10 may be positioned within the prepared acetabulum such that screw retention elements 20a, 20b, 20c, 20d, are aligned with ischium Is and pubis Pb of pelvis P, and screw retention elements 22a, 22b, 22c, of flange 26 are aligned with ilium Il of pelvis P. After acetabular cup 10 is properly aligned, a surgeon may press-fit the prosthesis into the prepared acetabulum of pelvis P, such as by using an impaction tool or a ball pusher, for example. Acetabular cup 10 may then be secured to pelvis P, such as by inserting bone fixation screw 30 into pelvis P.

The present invention provides a method of stabilizing acetabular cup 10 in pelvis P during securement, such as during insertion of bone fixation screw 30. In general, the method involves inserting at least one pin, such as a Kirschner wire or another suitable pin, into a location of pelvis P that is above acetabular cup 10 and radially opposite from a force applied during screw insertion. This method may prevent acetabular cup 10 from lifting away from pelvis P during screw insertion. The pins may be driven into pelvis P with a mallet, for example. Also, a suitable orthopaedic tool, such as tool 40 or tool 40' described below, may be used to stabilize acetabular cup 10 in pelvis P during securement.

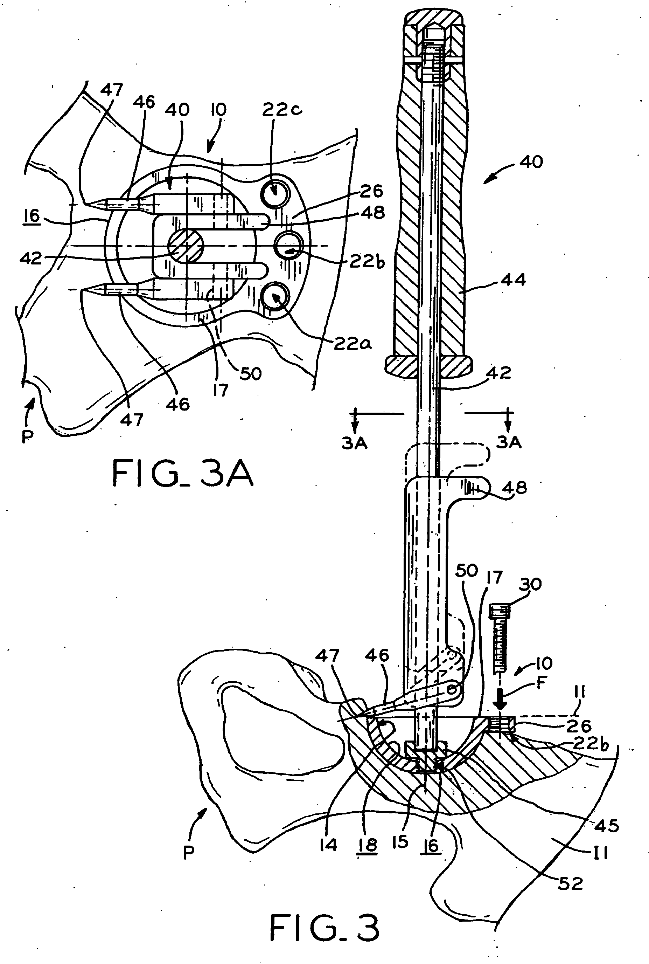

Referring to Figures 3 and 3A, a first embodiment of tool 40 is provided for stabilizing acetabular cup 10 in pelvis P during securement. Tool 40 includes shaft 42, handle 44 coupled to an end of shaft 42, and impactor or projection 45 coupled to shaft 42 at an end opposite of handle 44.

Tool 40 further includes at least one pin 46 coupled to shaft 42. In the illustrated embodiment, tool 40 includes two pins 46 extending in parallel. As pins 46 are moved into a locking position, pins 46 extend substantially transversely, or non-parallel, to shaft 42. Each pin 46 may include a sharp, pointed end 47, that is configured to be driven into bone. For example, end 47 of each pin 46 may include a pointed Kirschner wire or another suitable device that is configured to be driven into bone.

As shown in Figure 3, pins 46 are coupled to arm 48. Specifically, pins 46 are pivotally coupled to arm 48 at pivot points 50. Pins 46 may rotate independently or may be secured together for simultaneous rotation relative to arm 48. Arm 48 may include stops (not shown) to limit the rotational movement of pins 46 about arm 48. Arm 48 is slidingly coupled to shaft 42, such that arm 48 may be moved upward relative to shaft 42 toward handle 44 or downward relative to shaft 42 toward projection 45. Arm 48 may also be rotationally coupled to shaft 42.

In use, tool 40 may be pressed against acetabular cup 10. As shown in Figure 3, while gripping handle 44, a surgeon may press projection 45 of tool 40 into a corresponding bore 52 in articulating surface 18 of acetabular cup 10. It is within the scope of the present invention that projection 45 of tool 40 and bore 52 of acetabular cup 10 may be threaded to provide a fixed connection between the components. Alternatively, while gripping handle 44, a surgeon may press tool 40 against rim 17 of acetabular cup 10 or another suitable portion of acetabular cup 10. For example, tool 40 may include fingers (not shown) that extend radially from shaft 42 to engage rim 17 of acetabular cup 10. In this alternative embodiment, acetabular cup 10 need not include bore 52. In addition to potentially driving acetabular cup 10 into the prepared acetabulum of pelvis P, the force applied to tool 40 may stabilize acetabular cup 10 during screw insertion, as described below. In this position, shaft 42 of tool 40 may extend along longitudinal axis 15 of acetabular cup 10.

Next, the surgeon may align ends 47 of pins 46 with bone surrounding acetabular cup 10. Ends 47 of pins 46 may be aligned with bone that is located radially opposite from a desired screw retention element 20, 22. As shown in phantom in Figure 3, ends 47 of pins 46 are aligned with bone located above equatorial region 14 of acetabular cup 10, specifically bone located above rim 17 of acetabular cup 10. Ends 47 of pins 46 may extend within an outer periphery of acetabular cup 10 defined by exterior bone-contacting surface 16. Pins 46 may rest against equatorial region 14 of acetabular cup 10 to stabilize pins 46. Aligning pins 46 may involve raising or lowering arm 48 of tool 40 relative to shaft 42.

After pins 46 are properly aligned with the bone of pelvis P, the surgeon may force arm 48 downward relative to shaft 42 toward projection 45 of tool 40 and toward equatorial plane 11 of acetabular cup 10, as shown in solid in Figure 3. Pins 46 may contact equatorial region 14 of acetabular cup 10, such that forcing arm 48 downward causes pins 46 to rotate relative to arm 48 about pivot points 50. As the distance between pivot points 50 and equatorial plane 11 of acetabular cup 10 decreases, ends 47 of pins 46 protrude beyond the outer periphery of acetabular cup 10 defined by exterior bone-contacting surface 16 and into the bone of pelvis P. As arm 48 is forced downward, pins 46 may approach an essentially perpendicular position relative to shaft 42 of tool 40.

With pins 46 secured in the bone, bone fixation screws 30 may be driven through screw retention elements 20, 22, and into the bone of pelvis P. For example, as shown in Figure 3, bone fixation screw 30 may be driven through screw retention element 22b in flange 26 of acetabular cup 10 and into ilium Il of pelvis P.

Tool 40 is provided to stabilize acetabular cup 10 in pelvis P during screw insertion. As discussed above, screw retention elements 20, 22, may be radially offset from longitudinal axis 15 of acetabular cup 10, which is assumed to generally coincide with the center of gravity of acetabular cup 10. Therefore, applying a force to screw retention elements 20, 22, such as when tightening bone fixation screw 30 in place, may cause acetabular cup 10 to lift out of the prepared acetabulum of pelvis P. The force F applied to acetabular cup 10 when tightening bone fixation screw 30 in screw retention element 22b is illustrated schematically in Figure 3. As the distance between screw retention elements 20, 22, and longitudinal axis 15 of acetabular cup 10 increases, the magnitude of force F required to lift acetabular cup 10 decreases. For example, if external screw retention elements 20, 22, are provided about the periphery of acetabular cup 10, outside of articulating surface 18 of acetabular cup 10, the magnitude of force F applied when tightening bone fixation screw 30 may be sufficient to lift acetabular cup 10 away from pelvis P. A surgeon holding handle 44 may force projection 45 of tool 40 against articulating surface 18 of acetabular cup 10 to prevent acetabular cup 10 from lifting away from pelvis P. Additionally, the fixation between pins 46 of tool 40 and the bone of pelvis P that is located radially opposite from force F may prevent acetabular cup 10 from lifting away from pelvis P. Specifically, with pins 46 of tool 40 secured in place above acetabular cup 10, acetabular cup 10 may be prevented from moving upward beyond pins 46.

After inserting bone fixation screw 30 through acetabular cup 10 and into pelvis P, pins 46 of tool 40 may be removed from pelvis P and rotated in preparation for inserting another screw. According to an exemplary embodiment of the present invention, pins 46 of tool 40 may be rotated toward the bone of pelvis P that is located radially opposite from the next screw retention element 20, 22. In one embodiment, with projection 45 of tool 40 secured in bore 52 of acetabular cup 10, arm 48 of tool 40 may be rotated relative to shaft 42 to rotate pins 46. In another embodiment, projection 45 of tool 40 may be removed from bore 52 of acetabular cup 10, and handle 44 of tool 40 may be rotated to rotate pins 46.

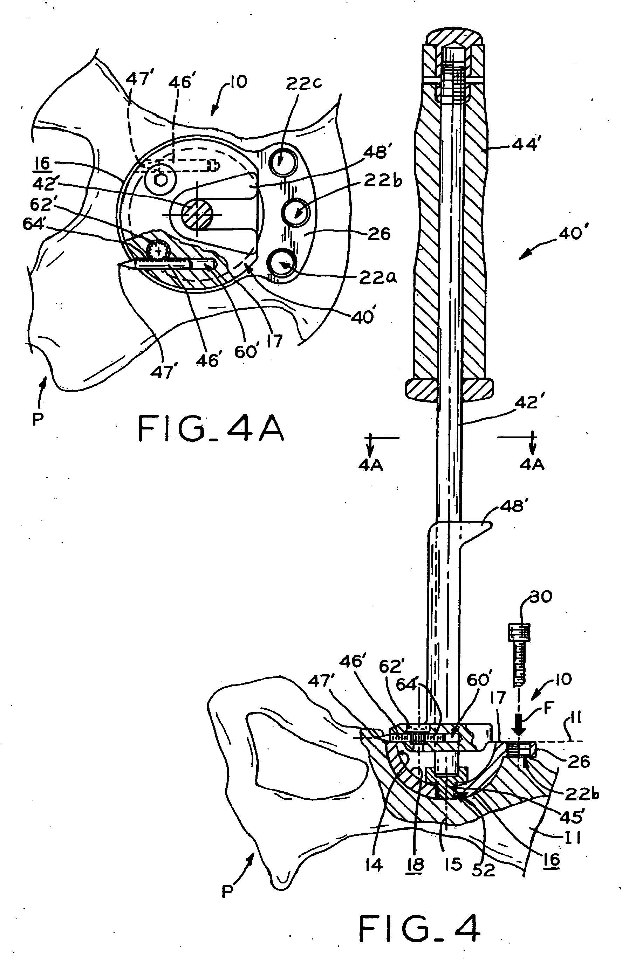

Referring next to Figure 4, tool 40' is provided for stabilizing acetabular cup 10 in pelvis P during securement. Except as described below, tool 40' shown in Figures 4-4A includes many elements that are identical or substantially identical to those of tool 40 shown in Figures 3-3A, and the same reference numerals followed by a prime symbol are used to designate identical or substantially identical elements therebetween. Tool 40' includes shaft 42', handle 44' coupled to an end of shaft 42', and projection 45' coupled to shaft 42' at an end opposite of handle 44'.

Tool 40' further includes at least one pin 46' coupled to shaft 42'. In the illustrated embodiment, tool 40' includes two pins 46' extending in parallel. Pins 46' extend essentially perpendicular to shaft 42'. Each pin 46' may include a sharp, pointed end 47', that is configured to be driven into bone. For example, end 47' of each pin 46' may include a Kirschner wire or another suitable device that is configured to be driven into bone.

As shown in Figure 4, pins 46' are coupled to arm 48'. Arm 48' may be coupled to shaft 42' to resist translational movement, or arm 48' may be slidingly coupled to shaft 42' such that arm 48' may be moved upward relative to shaft 42' toward handle 44' or downward relative to shaft 42' toward projection 45'. Arm 48' may also be rotationally coupled to shaft 42'. Arm 48' includes at least one chamber 60'. Pins 46' are received in chamber 60' and are configured to translate side-to-side in chamber 60'. In the illustrated embodiment of Figure 4, chamber 60' includes pinions 62' and pins 46' include racks 64' that engage pinions 62', such that rotation of pinions 62' causes pins 46' to translate in chamber 60'.

In use, tool 40' may be pressed against acetabular cup 10. As shown in Figure 4, while gripping handle 44', a surgeon may press projection 45' of tool 40' into a corresponding bore 52 in articulating surface 18 of acetabular cup 10. It is within the scope of the present invention that projection 45' of tool 40' and bore 52 of acetabular cup 10 may be threaded to provide a fixed connection between the components.
Alternatively, while gripping handle 44', a surgeon may press tool 40' against rim 17 of acetabular cup 10 or another suitable portion of acetabular cup 10. For example, tool 40' may include fingers (not shown) that extend radially from shaft 42' to engage rim 17 of acetabular cup 10. In this alternative embodiment, acetabular cup 10 need not include bore 52. In addition to potentially driving acetabular cup 10 into the prepared acetabulum of pelvis P, the force applied to tool 40' may stabilize acetabular cup 10 during screw insertion, as described below. In this position, shaft 42' of tool 40' may extend along longitudinal axis 15 of acetabular cup 10.

Next, the surgeon may align ends 47' of pins 46' with bone surrounding acetabular cup 10. Ends 47' of pins 46' may be aligned with bone that is located radially opposite from a desired screw retention element 20, 22. As shown in phantom in Figure 4A, end 47' of pin 46' is aligned with bone located above equatorial region 14 of acetabular cup 10, specifically bone located above rim 17 of acetabular cup 10. Ends 47' of pins 46' may extend within an outer periphery of acetabular cup 10 defined by exterior bone-contacting surface 16. Pins 46' may rest against equatorial region 14 of acetabular cup 10 to stabilize pins 46'. Aligning pins 46' may involve raising or lowering arm 48' of tool 40', if applicable.

After pins 46' are properly aligned with the bone of pelvis P, the surgeon may drive ends 47' of pins 46' beyond the outer periphery of acetabular cup 10 defined by exterior bone-contacting surface 16 and into the bone of pelvis P, as shown in solid in Figure 4A. The surgeon may drive ends 47' of pins 46' into the bone by rotating pinions 62' by hand or using a suitable tool, such as a hex wrench, for example. As discussed above, pinions 62' engage racks 64' of pins 46' to translate pins 46' in chamber 60'.

With pins 46' secured in the bone, bone fixation screws 30 may be driven through screw retention elements 20, 22, and into the bone of pelvis P. For example, as shown in Figure 4, bone fixation screw 30 may be driven through screw retention element 22b in flange 26 of acetabular cup 10 and into ilium Il of pelvis P.

Like tool 40, tool 40' is provided to stabilize acetabular cup 10 in pelvis P during screw insertion. The force F applied to acetabular cup 10 when tightening bone fixation screw 30 in screw retention element 22b is illustrated schematically in Figure 4. A surgeon holding handle 44' may force projection 45' of tool 40' against articulating surface 18 of acetabular cup 10 to prevent acetabular cup 10 from lifting away from pelvis P. Additionally, the fixation between pins 46' of tool 40' and the bone of pelvis P that is located radially opposite from force F may prevent acetabular cup 10 from lifting away from pelvis P. Specifically, with pins 46' of tool 40' secured in place above acetabular cup 10, acetabular cup 10 may be prevented from moving upward beyond pins 46'.

After inserting a bone fixation screw 30 through acetabular cup 10 and into pelvis P, pins 46' of tool 40' may be removed from pelvis P and rotated in preparation for inserting another screw. According to an exemplary embodiment of the present invention, pins 46' of tool 40' may be rotated toward the bone of pelvis P that is located radially opposite from the next screw retention element 20, 22. In one embodiment, with projection 45' of tool 40' secured in bore 52 of acetabular cup 10, arm 48' of tool 40' may be rotated relative to shaft 42' to rotate pins 46'. In another embodiment, projection 45' of tool 40' may be removed from bore 52 of acetabular cup 10, and handle 44' of tool 40' may be rotated to rotate pins 46'.

While this invention has been described as having exemplary designs, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. An orthopaedic tool for implanting a prosthetic socket, the prosthetic socket including a substantially hemispherical body having a convex exterior surface defining an outer periphery of the prosthetic socket, a concave interior articulating surface, and a rim extending between the convex exterior surface and the concave interior articulating surface near a top portion of the prosthetic socket,
**characterized in that**
the orthopaedic tool comprises:
a shaft configured to couple to the prosthetic socket; and
a first pin having an end, the first pin coupled to the shaft for movement relative to the shaft above the rim of the prosthetic socket from a first position in which the end of the first pin is located within the outer periphery of the prosthetic socket to a second position in which the end of the first pin is located beyond the outer periphery of the prosthetic socket.

2. The orthopaedic tool of claim 1, **characterized in that** the first pin is configured to at least one of translate and rotate relative to the shaft of the orthopaedic tool.

3. The orthopaedic tool of claim 1 or 2, **characterized in that** the first pin extends substantially transversely to the shaft in the second position.

4. The orthopaedic tool of any of claims 1 to 3, **characterized in that** a second pin is provided that extends substantially parallel to the first pin.

5. The orthopaedic tool of any of claims 1 to 4, **characterized in that** an arm is provided which is coupled to the shaft for at least one of translational and rotational movement relative to the shaft, the first pin coupled to the arm.

6. The orthopaedic tool of any of claims 1 to 5, **characterized in that** an arm is provided which is rotatably coupled to the shaft, the arm defining a chamber that is sized to receive the first pin.

7. An orthopaedic system
**characterized by**:
a prosthetic socket comprising a substantially hemispherical body that defines a polar region and an equatorial region located above the polar region, the body comprising:
a convex exterior surface that defines an outer periphery of the prosthetic socket;
a concave interior articulating surface; and
a longitudinal axis; and a tool comprising:
a shaft configured to couple to the prosthetic socket; and
a first pin having an end, the first pin coupled to the shaft for movement relative to the shaft above the equatorial region of the prosthetic socket from a first position in which the end of the first pin is located within the outer periphery of the prosthetic socket to a second position in which the end of the first pin is located beyond the outer periphery of the prosthetic socket.

8. The orthopaedic system of claim 7, **characterized in that** the shaft of the tool extends essentially parallel to the longitudinal axis of the prosthetic socket and is configured to couple to the concave interior articulating surface of the prosthetic socket.

9. The orthopaedic system of claim 7 or 8, **characterized in that** the concave interior articulating surface of the prosthetic socket comprises a bore that is sized to receive the shaft of the tool.

10. The orthopaedic system of any of claims 7 to 9, **characterized in that** the first pin is configured to at least one of translate and rotate relative to the shaft of the tool, and/or
that the first pin extends substantially transversely to the shaft in the second position.

11. The orthopaedic system of any of claims 7 to 10, **characterized in that** the tool further comprises a second pin that extends substantially parallel to the first pin.

12. The orthopaedic system of any of claims 7 to 11, **characterized in that** an arm is provided which is coupled to the shaft for at least one of translational and rotational movement relative to the shaft, the first pin coupled to the arm, and/or
that the tool further comprises an arm rotatably coupled to the shaft, the arm defining a chamber that is sized to receive the first pin.

13. The orthopaedic system of any of claims 7 to 12, **characterized in that** the prosthetic socket further comprises at least one screw retention element radially offset from the longitudinal axis.

14. The orthopaedic system of any of claims 7 to 13, **characterized in that** the prosthetic socket further comprises at least one screw retention element located outside of the concave interior articulating surface of the prosthetic socket.

15. The orthopaedic system of any of claims 7 to 14, **characterized in that** the prosthetic socket comprises one of an acetabular component and a glenoid component.
